# EUROPEAN PATENT SPECIFICATION

(11) **EP 2 586 769 B1**
(45) Date of publication and mention of the grant of the patent: **16.01.2019**
(21) Application number: 11795627.6
(22) Date of filing: 08.06.2011
(51) Int. Cl.: C07C 227/34, C07B 57/00, C07C 229/48

(54) **METHOD FOR PRODUCING (1R, 2S)-1-AMINO-2-VINYL CYCLOPROPANE CARBOXYLIC ACID ESTER THAT HAS IMPROVED OPTICAL PURITY**
VERFAHREN ZUR HERSTELLUNG VON (1R, 2S)-1-AMINO-2-VINYLCYCLOPROPAN-CARBONSÄUREESTERN VON VERBESSERTER OPTISCHER REINHEIT
PROCÉDÉ DE PRODUCTION D'ESTER DE L'ACIDE (1R, 2S)-1-AMINO-2-VINYL- CYCLOPROPANE CARBOXYLIQUE DE PURETÉ OPTIQUE AMÉLIORÉE

(30) Priority: 15.06.2010 JP 2010136377
(43) Date of publication of application: 01.05.2013
(73) Proprietor: KANEKA CORPORATION, Osaka (JP)
(72) Inventor: TANAKA, Tatsuyoshi, Takasago-shi Hyogo 676-8688 (JP); NISHIYAMA, Akira, Takasago-shi Hyogo 676-8688 (JP)
(74) Representative: Hoffmann Eitle
(86) International application number: PCT/JP2011/063181
(87) International publication number: WO 2011/158720

(56) References cited:
- WO-A1-2008/099730
- WO-A1-2008/099730
- JP-A- 2010 510 233
- Pierre L Beaulieu ET AL: "Synthesis of (1R,2S)-1-Amino-2-Vinylcyclopropane Carboxylic Acid (Vinyl-ACCA) Derivatives: Key Intermediates for the Preparation of Inhibitors of the Hepatitis C Virus NS3 Protease Supporting Information", , 17 June 2005 (2005-06-17), XP055215264, Retrieved from the Internet: URL:http://pubs.acs.org/doi/suppl/10.1021/ jo050468q/suppl_file/jo050468qsi20050513_0 10841.pdf [retrieved on 2015-09-22]
- P.L.BEAULIEU ET AL.: 'Synthesis of (1R,2S)-1-Amino-2-vinylcyclopropanecarboxyl ic Acid Vinyl-ACCA) Derivatives:Key Intermediates for the Preparation of Inhibitors of the Hepatitis C Virus NS3 Protease' J.ORG.CHEM. vol. 70, no. 15, 2005, pages 5869 - 5879, XP002686607

## Description

### TECHNICAL FIELD

The present invention relates to a method for producing a (1R, 2S)-1-amino-2-vinylcyclopropanecarboxylic acid ester, which is useful for drugs, especially for an intermediate of a Hepatitis C drug.

### BACKGROUND ART

The following methods for producing a (1R, 2S)-1-amino-2-vinylcyclopropanecarboxylic acid ester are known;
Conventional art 1: Non-Patent Document 1 discloses a method for producing a mixture of (1S, 2R)-isomer and (1R, 2S)-isomer of a 1-amino-2-vinylcyclopropanecarboxylic acid ethyl ester in preference to a mixture of (1S, 2S)-isomer and (1R, 2R)-isomer, by reacting a benzylidene glycine ethyl ester with 1,4-dibromobutene in the presence of lithium tert-butoxide. The product is a racemic mixture containing (1S, 2R)-isomer and (1R, 2S)-isomer. Thus, by performing a selective-hydrolysis of the enantiomer ((1S, 2R)-isomer) using an alcalase, the (1R, 2S)-isomer is obtained (Non-Patent Document 1);
Conventional art 2: Non-Patent Document 1 also discloses a method of chiral resolution of a racemic 1-amino-2-vinylcyclopropanecarboxylic acid ethyl ester using di-p-toluoyl-D-tartaric acid;
Conventional art 3: Non-Patent Document 2 discloses a method for producing a (1R, 2S)-1-amino-2-vinylcyclopropanecarboxylic acid ester by an asymmetric cyclopropanation with coupling a benzylidene glycine ethyl ester and 1,4-dibromobutene in the presence of a chiral phase-transfer catalyst derived from cinchona alkaloid. The optical purity thereof is improved by supercritical fluid chromatography (SFC).

### PRIOR ART

### Non-Patent Document

Non-Patent Document 1: J. Org. Chem., 2005, 70(15), 5869-5879.
Non-Patent Document 2: Organic Process Research & development, 2010, 14, 692-700.

### DISCLOSURE OF THE INVENTION

### PROBLEMS TO BE SOLVED BY THE INVENTION

In the conventional art 1, there is a problem on productivity, since a substrate concentration when performing the asymmetric hydrolysis using alcalase is low. Additionally, it takes 3 days to complete the hydrolysis. Thus, there is a difficulty in producing them on an industrial scale. Further, with regard to the conventional art 2, even if a crystallization is repeated twice, the optical purity only goes up to 50-55 %ee. Thus, the method is impractical in this regard. In the conventional art 3, the optical purity of the product obtained by the asymmetric cyclopropanation is at most around 80%ee. Therefore, another production method to achieve a higher optical purity is further required. Additionally, in the conventional art 3, there is a problem to need a very special facility such as a supercritical fluid chromatography for improving the optical purity.

### MEANS FOR SOLVING THE PROBLEMS

As a result of an earnest study, the present inventors discovered an efficient method for chiral resolution of racemic trans-1-amino-2-vinylcyclopropanecarboxylic acid ester or (1R, 2S)-1-amino-2-vinylcyclopropanecarboxylic acid ester having low optical purity, and a method for improving the optical purity thereof; and accomplished the present invention.

[0006]-[0009] The present invention relates to a method for producing a salt represented by the formula (3) comprising the steps of:
forming the salt represented by the following formula (3): 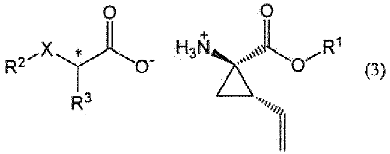 wherein, R¹ represents a C₁₋₅ alkyl group; R² represents a hydrogen atom, a C₁₋₁₅ alkyl group, a C₆₋₁₅ aryl group, a C₇₋₁₅ aralkyl group, a C₂₋₁₅ acyl group, a C₇₋₁₅ aroyl group or a C₇₋₁₅ N-arylcarbamoyl group; R³ represents a C₁₋₁₅ alkyl group, a C₆₋₁₅ aryl group or a C₇₋₁₅ aralkyl group; X represents O, S, or NH; * indicates an asymmetric carbon atom,
from (1R, 2S)-1-amino-2-vinylcyclopropanecarboxylic acid ester containing (1S, 2R)-1-amino-2-vinylcyclopropanecarboxylic acid ester as an impurity and represented by the following formula (1) : 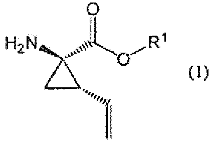 wherein, R¹ is same as the above,
and an optically active carboxylic acid represented by the following formula (2): 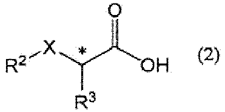 wherein, R² , R³ , X and * are same as the above,
and precipitating the salt (3) from a solvent as a solid to remove the impurity.

The present invention is applicable to a method for producing a trans-1-amino-vinylcyclopropanecarboxylic acid ester improved in optical purity, which comprises the steps of,
contacting a solution of a raw material, i.e. a trans-1-amino-2-vinylcyclopropanecarboxylic acid ester, with the optically active carboxylic acid represented by the formula (2),
precipitating the salt which is made from the trans-1-amino-2-vinylcyclopropanecarboxylic acid ester and the optically active carboxylic acid,
separating the precipitated salt from the solution, and dissociating the salt.

[0011]-[0012] The present invention also relates to (1R, 2S)-1-amino-2-vinylcyclopropanecarboxylic acid ester derivative represented by the following formula (4): 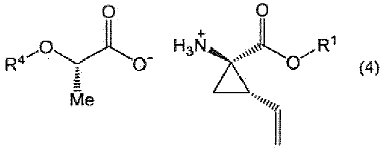 wherein R¹ represents a C₁₋₅ alkyl group; R⁴ represents a C₇₋₁₅ aroyl group.

### EFFECT OF THE INVENTION

According to the method of the present invention, (1R, 2S)-1-amino-2-vinylcyclopropanecarboxylic acid ester can be efficiently produced at low cost.

### MODE FOR CARRYING OUT THE INVENTION

There are two types of isomers in 1-amino-2-vinylcyclopropanecarboxylic acid ester; one isomer is a trans-isomer (i.e., (1R, 2S)-isomer, (1S, 2R)-isomer), in which a vinyl group and an amino group exist on the opposite side across the plane of the cyclopropane ring; and the other isomer is a cis-isomer (i.e., (1R, 2R)-isomer, (1S, 2S)-isomer), in which a vinyl group and an amino group exist on the same side. According to the present invention, it is possible to improve an optical purity of the trans-isomers. In the following description, the present invention is described by taking an example where an optical purity of (1R, 2S)-isomer, which is one trans-isomer, can be improved by using a specic optically active carboxylic acid. Needless to say, however, the following method is also applicable to the method for improving the optical purity of (1S, 2R)-isomer, the other trans-isomer, if an optically active carboxylic acid having an opposite optical rotation to the optically active carboxylic acid described below is used.

[0015]-[0017] The (1R, 2S)-1-amino-2-vinylcyclopropanecarboxylic acid ester which is a starting material for the present invention is represented by the following formula (1): 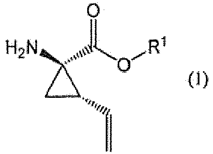 wherein R¹ is a C₁₋₅ alkyl group, and preferably C₁₋₃ alkyl group. R¹ is specifically exemplified by a methyl group, an ethyl group, an n-propyl group, an isopropyl group, an n-butyl group, an isobutyl group, a sec-butyl group, a tert-butyl group and the like; preferably a methyl group or an ethyl group, and more preferably an ethyl group.

The raw material compound (1) has low optical purity and contains an enantiomer, i.e. a (1S, 2R)-1-amino-2-vinylcyclopropanecarboxylic acid ester, as an impurity. For example, the raw material containing both enantiomers (1S, 2R)-isomer and (1R, 2S)-isomer in equal molar amounts, i.e. a racemic 1-amino-2-vinylcyclopropanecarboxylic acid ester, can be produced by the process described in Non-Patent Document 1. Additionally, for example, the raw material containing the (1S, 2R)-isomer, which is the enantiomer of the (1R, 2S)-isomer, in less than equal molar amount, i.e. a (1R, 2S)-1-amino-2-vinylcyclopropanecarboxylic acid ester having low optical purity, can be produced by the process described in Non-Patent Document 2.

More specifically, a racemic 1-amino-2-vinylcyclopropanecarboxylic acid ester can be produced by coupling N-methylene glycine ester with trans-1,4-dihalo-2-butene in the presence of a base for cyclopropanation, and removing the N-methylene group (imine) by hydrolysis. In the above steps, the N-methylene glycine ester is exemplified by N-arylmethylene glycine ester such as N-phenylmethylene glycine ethyl ester; the trans-1,4-dihalo-2-butene is exemplified by trans-1,4-dibromo-2-butene; the base is particularly exemplified by a base having a bulky group such as a trialkylmethyl group and a trialkylsilyl group, and specifically exemplified by lithium tert-butoxide, lithium hexamethyldisilazane, and sodium hexamethyldisilazane.

The (1R, 2S)-isomer having low optical purity (specifically, approximately not more than 84%ee, for example, approximately in the range of 1 to 80%ee, particularly approximately in the range of 1 to 55%ee) can also be produced by coupling reaction of N-methylene glycine ester and a trans-1,4-dihalo-2-butene for cyclopropanation, as is the case for the racemic mixture. In case of producing the (1R, 2S)-isomer having low optical purity, the above reaction is performed with use of a chiral catalyst in the presence of a solid base such as a sodium hydroxide powder. The chiral catalyst is exemplified by a quaternary ammonium salt, i.e. cinchoninium salt, which is obtained by quaternization of a nitrogen atom at a bridgehead of cinchonine with an organic group (preferably an arylmethyl group such as a benzyl group, a naphthylmethyl group, an anthracenylmethyl group).

[0021]-[0022] The racemic raw material or the (1R, 2S)-isomer having low optical purity can be usually improved in the optical purity by dissolving the compound in an appropriate solvent and then precipitating the (1R, 2S)-isomer from the solution. The present invention is characterized in that the (1R, 2S)-isomer is reacted with the optically active carboxylic acid to be a salt, and then the salt is precipitated.

The optically active carboxylic acid used for the present invention is represented by the following formula (2):

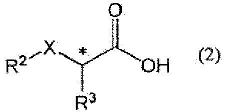

In the formula, R² is a hydrogen atom; a C₁₋₁₅ alkyl group, preferably a C_{1-*10} alkyl group, more preferably a C₁₋₅ alkyl group; an aromatic ring group such as a C₆₋₁₅ aryl group, preferably a C₆₋₉ aryl group, or a hydrogenated group thereof; an alkyl group bonded with an aromatic ring, such as a C₇₋₁₅ aralkyl group, preferably a C₇₋₁₀ aralkyl group, or a hydrogenated group thereof; a C₂₋₁₅ acyl group, preferably a C₂₋₅ acyl group; an acyl group bonded with an aromatic ring such as a C₇₋₁₅ aroyl group, preferably C₇₋₁₀ aroyl group, or a group obtained by hydrogenation of the aromatic ring thereof; a carbamoyl group bonded with an aromatic ring to the N atom such as a C₇₋₁₅ N-arylcarbamoyl group, preferably a C₇₋₁₀ N-arylcarbamoyl group, or a group obtained by hedrogenation of the aromatic ring thereof. R² is preferably an aroyl group, a carbamoyl group and the like. The above groups as R² may have 1 or more substituent groups. The substituent group is exemplified by an alkyl group, a halogenated alkyl group, an alkoxy group, a halo group (particularly, a chloro group, a bromo group), a nitro group, a hydroxy group and the like; preferably, a group having halogen atom, such as a halogenated alkyl group and a halo group.

R² is specifically a hydrogen atom; an acyclic alkyl group such as a methyl group, an ethyl group, an n-propyl group, an isopropyl group, an n-butyl group, an isobutyl group, a sec-butyl group, a tert-butyl group, an n-pentyl group; a cyclic alkyl group such as a cyclopropyl group, a cyclobutyl group, a cyclohexyl group and an adamantyl group; a substituted or an unsubstituted phenyl group such as a phenyl group, a p-chlorophenyl group, an m-(trifluoromethyl)phenyl group, a p-methoxyphenyl group; a substituted or unsubstituted naphthyl group such as a 1-naphthyl group; a phenylalkyl group such as a benzyl group, a 1-phenethyl group, a diphenylmethyl group, a triphenylmethyl group; a C₁₋₅ alkylcarbonyl group such as an acetyl group, an isobutyryl group, a pivaloyl group; a substituted or an unsubstituted benzoyl group such as a benzoyl group, a p-methylbenzoyl group, a p-nitrobenzoyl group, a p-methoxybenzoyl group, an o-chlorobenzoyl group, an m-chlorobenzoyl group, a p-chlorobenzoyl group, a 3,5-dichlorobenzoyl group, a 3,4-dichlorobenzoyl group, a p-bromobenzoyl group, a 4-phenylbenzoyl group; a substituted or an unsubstituted naphthoyl group such as a 1-naphthoyl group; N-phenylcarbamoyl group and the like. Preferably, R² is a phenyl group, a p-chlorophenyl group, a m-(trifluoromethyl)phenyl group, a p-methoxyphenyl group, a benzoyl group, a p-methylbenzoyl group, a p-nitrobenzoyl group, a p-methoxybenzoyl group, an o-chlorobenzoyl group, an m-chlorobenzoyl group, a p-chlorobenzoyl group, a 3,5-dichlorobenzoyl group, a 3,4-dichlorobenzoyl group, a p-bromobenzoyl group, a 1-naphthoyl group, a 4-phenylbenzoyl group, N-phenylcarbamoyl group; more preferably a phenyl group, an m-(trifluoromethyl)phenyl group, a benzoyl group, a p-chlorobenzoyl group, a 3,5-dichlorobenzoyl group, N-phenylcarbamoyl group; particularly a p-chlorobenzoyl group, 3,5-dichlorobenzoyl group are preferred.

R³ is a C₁₋₁₅ alkyl group, preferably a C₁₋₁₀ alkyl group, more preferably a C₁₋₅ alkyl group; a C₆₋₁₅ aryl group, preferably a C₆₋₉ aryl group; a C₇₋₁₅ aralkyl group, preferably a C₇₋₁₀ aralkyl group. R³ groups may have 1 or more substituent groups. Such a substituent group is exemplified by an alkyl group, a halogenated alkyl group, an alkoxy group, a halo group (particularly, a chloro group, a bromo group), a nitro group, a hydroxy group and the like; preferably, a group having halogen atom such as a halogenated alkyl group or a halo group, and a hydroxy group.

R³ is specifically exemplified by an acyclic alkyl group such as a methyl group, an ethyl group, an n-propyl group, an isopropyl group, an n-butyl group, an isobutyl group, a sec-butyl group, a tert-butyl group, an n-pentyl group; a cyclic alkyl group such as a cyclopropyl group, a cyclobutyl group, a cyclohexyl group, an adamantyl group; a substituted or an unsubstituted phenyl group such as a phenyl group, an o-chlorophenyl group, a p-chlorophenyl group, an m-trifluoromethylphenyl group, a p-hydroxyphenyl group, a p-methoxyphenyl group; a substituted or an unsubstituted naphthyl group such as a 1-naphthyl group; a benzyl group, a 1-phenethyl group, a diphenylmethyl group, a triphenylmethyl group and the like. Preferably, R³ is a methyl group, a phenyl group, an o-chlorophenyl group, a p-chlorophenyl group, an m-(trifluoromethyl)phenyl group, a p-methoxyphenyl group, a p-hydroxyphenyl group, or a 1-naphthyl group; more preferably a methyl group, a p-chlorophenyl group, a p-hydroxyphenyl group; particularly, a methyl group is preferred.

The X represents O, S or NH, preferably O or NH, and more preferably O.

The symbol "*" indicates an asymmetric carbon atom. In the case of precipitating the (1R, 2S)-isomer, absolute configuration is preferably S. In contrast, in the case of precipitating the (1S, 2R)-isomer, the configuration of the carbon atom indicated by * is preferably R.

In case where the configuration of the carbon atom indicated by * carbon atom is S, the optically active carboxylic acid is preferably exemplified by (S)-α-phenoxy-p-chlorophenylacetic acid, (S)-α-[m-(trifluoromethyl)phenoxy]-p-chlorophenylacetic acid, (S)-benzoyllactic acid, (S)-p-chlorobenzoyllactic acid, (S)-3,5-dichlorobenzoyllactic acid and (S)-N-(N-phenylcarbamoyl)-p-hydroxyphenylglycine, and more preferably (S)-p-chlorobenzoyllactic acid and (S)-3,5-dichlorobenzoyllactic acid.

[0030]-[0031] The salt consisting of the (1R, 2S)-isomer and the optically active carboxylic acid (sometimes referred to as "a (1R, 2S)-1-amino-2-vinylcyclopropanecarboxylic acid ester derivative") is represented by the following formula (3): 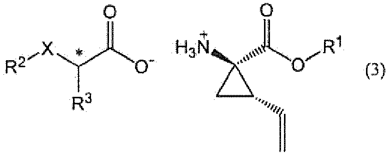 wherein, R¹, R², R³, X and * are same as the above.

[0032]-[0033] The compound (3) is preferably a (1R, 2S)-1-amino-2-vinylcyclopropanecarboxylic acid ester derivative represented by the following formula (4): 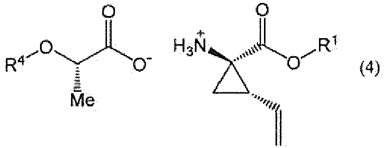 wherein R¹ is same as the above, R⁴ represents an optionally substituted C₇₋₁₅ aroyl group. R⁴ is specifically exemplified by a benzoyl group, a p-methylbenzoyl group, a p-nitrobenzoyl group, a p-methoxybenzoyl group, an o-chlorobenzoyl group, an m-chlorobenzoyl group, a p-chlorobenzoyl group, a 3,5-dichlorobenzoyl group, a 3,4-dichlorobenzoyl group, a p-bromobenzoyl group, a 1-naphthoyl group, a 4-phenylbenzoyl group and the like; preferably, a p-chlorobenzoyl group or a 3,5-dichlorobenzoyl group. In addition, the compound (4) is a novel compound which has not been described in any documents.

As mentioned above, a raw material (1R, 2S)-isomer is racemic or has low optical purity. In other words, the (1S, 2R)-isomer is contained as an impurity. Even when such a mixture containing the (1R, 2S)-isomer and the (1S, 2R)-isomer is used, by using the optically active carboxylic acid represented by the formula (2), it is possible to form the the salt of the (1R, 2S)-isomer and obtain the salt in the form of a solid.

The amount of the optically active carboxylic acid represented by the formula (2) (also referred to as "compound (2)") per 1 mole of the raw material (1R, 2S)-isomer (also referred to as "compound (1)") is preferably 0.1-2 mole, more preferably 0.5-1.5 mole, particularly, 0.5-1 mole is preferred. For example, 1 mole of the raw material (1R, 2S)-isomer corresponds to 1 mole of trans-1-amino-2-vinylcyclopropanecarboxylic acid ester, i.e. the sum of the (1R, 2S)-isomer and the (1S, 2R)-isomer.

For precipitating the solid, for example, the raw material (1R, 2S)-isomer (compound (1)) and the carboxylic acid (2) are mixed in a solvent as described below. The suitable solvent is not particularly limited, and specifically exemplified by water; an alcohol solvent such as methanol, ethanol, n-propanol, isopropanol, n-butanol, tert-butanol, ethylene glycol; an ester solvent such as ethyl acetate, n-propyl acetate, isopropyl acetate; an ether solvent such as tetrahydrofuran, diethyl ether, 1,4-dioxane, methyl tert-butyl ether, diisopropyl ether, ethylene glycol dimethyl ether; a ketone solvent such as acetone, methyl ethyl ketone; a nitrile solvent such as acetonitrile, propionitrile; an aromatic hydrocarbon solvent such as benzene, toluene, xylene; an aliphatic hydrocarbon solvent such as pentane, hexane, heptane, cyclohexane, methylcyclohexane; a halogenated solvent such as dichloromethane, 1,2-dichloroethane, chlorobenzene; a sulfoxide solvent such as dimethyl sulfoxide; an amide solvent such as N,N-dimethylformamide, N,N-dimethylacetamide; an urea solvent such as dimethylpropyleneurea; a phosphonic-triamide solvent such as hexamethylphosphonic triamide. One of the solvent may be used by itself, or plural solvents may be used in combination. If the plural solvents are used in combination, the mixing ratio thereof is not particularly limited.

The solvent is preferably water, methanol, ethanol, isopropanol, ethyl acetate, isopropyl acetate, tetrahydrofuran, methyl tert-butyl ether, diethyl ether, acetone, acetonitrile, toluene, chlorobenzene, hexane, heptane, cyclohexane, or methylcyclohexane; more preferably, ethyl acetate, methyl tert-butyl ether, toluene, hexane, heptane, cyclohexane; ethyl acetate, toluene or hexane is particularly preferred.

The use amount of the solvents is preferably not more than 50 parts by weight, and more preferably not more than 20 parts by weight relative to 1 parts by weight of the compound (1), since too much amount is not preferable in terms of cost and post processing.

The method of the present invention for precipitating the compound (3) as a solid is not particularly limited. For example, the following methods can be exemplified;
(a) a method of precipitating the solid by mixing the compound (1) and the carboxylic acid (2) in the above-described solvent;
(b) a method of precipitating the solid by mixing the compound (1) and the carboxylic acid (2) in the above-described solvent (particularly in a rich solvent) and then cooling the mixture;
(c) a method of precipitating the solid by mixing the compound (1) and the carboxylic acid (2) in the above-described solvent (particularly in a rich solvent) and then concentrating the mixture;
(d) a method of precipitating the solid by mixing the compound (1) and the carboxylic acid (2) in the above-described solvent (particularly in a rich solvent) and then further adding a poor solvent thereto;
(e) a method of precipitating the solid by mixing the compound (1) and the carboxylic acid (2) in the above-described solvent (particularly in a rich solvent), and replacing the solvent with a poor solvent.

Additionally, the methods (a) to (e) may be appropriately combined to precipitate the solid. Further, a solid used as a seed may be added for precipitating the solid.

The method is preferably a method of using at least a rich solvent like the methods (b) to (e); more preferably a method of using a rich solvent and a poor solvent in combination like the methods (d) or (e).

The rich solvent is exemplified by an alcohol solvent, an ester solvent, an ether solvent, a nitrile solvent, an aromatic hydrocarbon solvent, a sulfoxide solvent, an amide solvent, an urea solvent, a phosphonic-triamide solvent and the like; preferably an ester solvent, an ether solvent, an aromatic hydrocarbon solvent and the like; particularly an ester solvent, an aromatic hydrocarbon solvent.

As long as the solubility for the compound (3) is lower than that of the rich solvent to be used, the poor solvent can be arbitrarily selected from the above solvents, particularly the poor solvent can be preferentially selected from the solvents other than the rich solvent described above. In particular, an aliphatic hydrocarbon solvent is exemplified as a poor solvent which can be used at any time regardless of any rich solvents to be used. Particularly, hexane, heptane and the like are preferably exemplified as a poor solvent used in the methods (d) or (e).

The temperature for performing the methods (a) to (e) to precipitate the solid is not particularly limited, and may be appropriately set out depending on kinds of the salt to be formed and kinds of solvent to be used. Preferably, depending on the aimed precipitating amount and the aimed purity of the solid, the temperature may be set to be lower than the temperature the compound (3) can be dissolved in used solvent species or mixed solvent species. The temperature, for example, may be approximately -10 to 40°C.

The precipitated compound (3) obtained by the methods of precipitating solid, i.e. the methods (a) to (e), can be collected with separation using a method such as a suction filtration, a pressure filtration, a centrifugation or the like. Additionally, in case that a mother liquid remains in the obtained solid, the enantiomeric excess (%ee) of the solid might be degraded. In such a case, the quality may be improved as needed by further performing a wash with an organic solvent.

The method of drying the solid is not particularly limited; preferably the drying is performed at not more than about 60°C under reduced pressure or in vacuo to avoid a thermal decomposition and melting.

Additionally, in case that the enantiomeric excess degree (%ee) does not fully increase, the following method may be performed:
precipitating the solid again in either manner described in the methods (a) to (e);
washing the compound (3) with a solvent;
precipitating the solid again according to either manner based on the methods (a) to (e).

By dissociating the compound (3) obtained by the above method as needed, it is possible to obtain the compound (1) having an improved enantiomeric excess degree (%ee). The method of dissociating the salt, for example, may be performed in the manner comprising the steps of:
adding an alkaline solution to the compound (3) in order to make the compound (1) free from the salt,
extracting the compound using an organic solvents such as ethyl acetate, toluene, methyl tert-butyl ether, and
distilling the solvent used for the extraction by heating under reduced pressure.

The above alkaline solution is, for example, sodium hydroxide, potassium hydroxide, sodium carbonate, potassium carbonate, sodium hydrogen carbonate, potassium hydrogen carbonate. Additionally, a salt exchange may be carried out as follows to obtain the salt consisting of the compound (1) which is improved enantiomeric excess degree (%ee) and a strong acid by reacting the strong acid with the compound (3) obtained by the above method. The strong acid is exemiplified by hydrochloric acid, hydrobromic acid, sulfuric acid, methanesulfonic acid, p-toluenesulfonic acid or the like.

The compound (3) is a novel compound which has not been described in any documents, thus, it is unknown that the compound may become a solid. Further, the solid of the present invention means crystal, amorphous or the mixture thereof. The mixing ratio thereof is an optional matter.

According to the present invention described above, it is possible to improve the optical purity of a (1R, 2S)-1-amino-2-vinylcyclopropanecarboxylic acid ester contained in the compound (3), and also improve the optical purity of an (1R, 2S)-1-amino-2-vinylcyclopropanecarboxylic acid ester obtained after dissociating the salt thereof. The optical purity thereof, for example, may increase by not less than 5%ee, preferably not less than 30%ee, more preferably not less than 60%ee, and particularly not less than 80%ee, compared to the optical purity of a raw material. The optical purity of a (1R, 2S)-1-amino-2-vinylcyclopropanecarboxylic acid ester contained in the compound (3) or obtained after dissociating the salt thereof, for example, may be not less than 50%ee, preferably not less than 70%ee, more preferably not less than 85%ee, particularly not less than 90%ee.

### EXAMPLES

The present invention is explained in more detail by examples; however, the present invention is not limited by the examples.

Optical purity analysis - isocratic elusion
Column: DAICEL CHEMICAL CHIRALPAK AD-RH 150×4.6mm
Mobile phase: methanol/water = 2/1(capacity ratio)
Flow rate: 0.5 ml/min
Detector: UV220nm
Column temperature: 30°C
Retention time for (1R, 2S)-1-amino-2-vinylcyclopropanecarboxylic acid ethyl ester: 8.3 minutes
Retention time for (1S, 2R)-1-amino-2-vinylcyclopropanecarboxylic acid ethyl ester: 13.5 minutes

### Example 1: Production of (1R, 2S)-1-amino-2-vinylcyclopropanecarboxylic acid ethyl ester/(S)-p-chlorobenzoyl lactate;

[0051]-[0052]

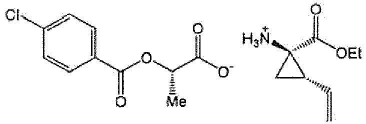

To the solution consisting of racemic 1-amino-2-vinylcyclopropanecarboxylic acid ethyl ester (210 mg (1 mmol), chemical purity: 70.4%), (S)-p-chlorobenzoyl lactic acid (229 mg (1 mmol)) and ethyl acetate (2 mL), hexane (2 mL) was added. As a result, a solid was precipitated. The mixture was stirred at 25°C for 3 hours, and then the solid was filtered under reduced pressure. The solid was washed with hexane (3 mL), and dried in a vacuum, thereby the white solid of the title compound was obtained (yield: 116mg, optical purity: 86.0%ee) .
¹H-NMR (CDCl₃) : δ (ppm) 1.26(t, 3H), 1.44 (dd, 1H), 1.57(dd, 1H), 1.58(d, 3H), 2.11(dd, 1H), 3.86(brs, 3H), 4.17(q, 2H) , 5.10(dd, 1H), 5.24(m, 1H), 5.67(m, 1H), 7.42(d, 2H), 8.00(d, 2H)

### Example 2: Production of (1R, 2S)-1-amino-2-vinylcyclopropanecarboxylic acid ethyl ester/(S)-p-chlorobenzoyl lactate

To the solution consisting of 1-amino-2-vinylcyclopropanecarboxylic acid ethyl ester with 52.3%ee (227 mg (1 mmol), chemical purity: 68.0%), (S)-p-chlorobenzoyl lactic acid (183 mg (0.8 mmol)) and ethyl acetate (1 mL), hexane (2 mL) was added. As a result, a solid was precipitated. The mixture was stirred at 5°C for 1 hour, and then the solid was filtered under reduced pressure. The solid was washed with hexane (2 mL), and dried in a vacuum, thereby the white solid of the title compound was obtained (yield: 223 mg, optical purity: 95.3%ee).

### Example 3: Production of (1R, 2S)-1-amino-2-vinylcyclopropanecarboxylic acid ethyl ester/(S)-3,5-dichlorobenzoyl lactate;

[0054]-[0055]

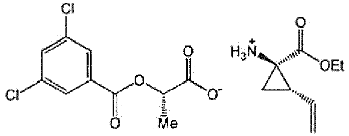

To the solution consisting of racemic 1-amino-2-vinylcyclopropanecarboxylic acid ethyl ester (210 mg (1 mmol), chemical purity: 70.4%), (S)-3,5-dichlorobenzoyl lactic acid (210 mg (0.8 mmol)) and toluene (1 mL), hexane (3 mL) was added. As a result, a solid was precipitated. The mixture was stirred at 5°C for 1 hour, and then the solid was filtered under reduced pressure. The solid was washed with hexane (3 mL), and dried in a vacuum, thereby the white solid of the title compound was obtained (yield: 93 mg, optical purity: 96.1%ee).
¹H-NMR (CDCl₃) : δ(ppm) 1.26(t, 3H), 1.49(dd, 1H), 1.59(dd, 1H), 1.61(d, 3H), 2.16(dd, 1H), 4.98(brs, 3H), 5.10(q, 2H), 5.22(m, 2H), 5.67(m, 1H), 7.54(s, 1H), 7.93(s, 2H)

### Example 4: Production of (1R, 2S)-1-amino-2-vinylcyclopropanecarboxylic acid ethyl ester/(S)-3, 5-dichlorobenzoyl lactate

To the solution consisting of 1-amino-2-vinylcyclopropanecarboxylic acid ethyl ester with 52.3%ee (285 mg (1.25 mmol), chemical purity: 68.0%), (S)-3,5-dichlorobenzoyl lactic acid (263 mg (1 mmol)) and toluene (1 mL), hexane (3 mL) was added. As a result, a solid was precipitated. The mixture was stirred at 5°C for 1 hour, and then the solid was filtered under reduced pressure. The solid was washed with hexane (2 mL), and dried in a vacuum, thereby the white solid of the title compound was obtained (yield: 322 mg, optical purity: 91.7%ee).

### Example 5: Production of (1R, 2S)-1-amino-2-vinylcyclopropanecarboxylic acid ethyl ester/(S)-benzoyl lactate;

[0057]-[0058]

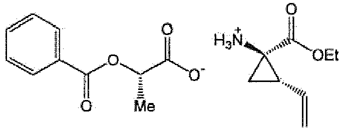

To the solution consisting of racemic 1-amino-2-vinylcyclopropanecarboxylic acid ethyl ester (210 mg (1 mmol), chemical purity: 70.4%), (S)-benzoyl lactic acid (194 mg (1 mmol)) and ethyl acetate (1 mL), hexane (5 mL) was added. As a result, a solid was precipitated. The mixture was stirred at 25°C for 1 hour, and then the solid was filtered under reduced pressure. The solid was washed with hexane (3 mL), and dried in a vacuum, thereby the white solid of the title compound was obtained (yield: 37 mg, optical purity: 82.3%ee).

### Example 6: Production of (1R, 2S)-1-amino-2-vinylcyclopropanecarboxylic acid ethyl ester/(S)-α-phenoxy-p-chlorophenyl acetate;

[0059]-[0060]

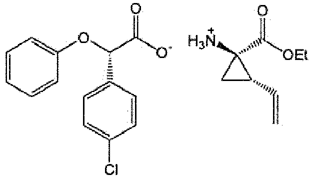

To the solution consisting of racemic 1-amino-2-vinylcyclopropanecarboxylic acid ethyl ester (210 mg (1 mmol), chemical purity: 70.4%), (S)-α-phenoxy-p-chlorophenyl acetic acid (262 mg (1 mmol)) and ethyl acetate (1 mL), hexane (2 mL) was added. As a result, a solid was precipitated. The mixture was stirred at 5°C for 1 hour, and then the solid was filtered under reduced pressure. The solid was washed with hexane (2 mL), and dried in a vacuum, thereby the white solid of the title compound was obtained (yield: 79 mg, optical purity: 75.5%ee).

### Example 7: Production of (1R, 2S)-1-amino-2-vinylcyclopropanecarboxylic acid ethyl ester/(S)-α-[m-(trifluoromethyl)phenoxy]-p-chlorophenyl acetate;

[0061]-[0062]

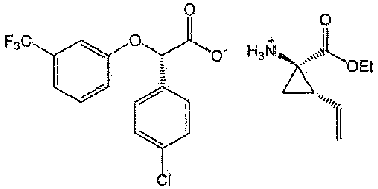

To the solution of consisting of racemic 1-amino-2-vinylcyclopropanecarboxylic acid ethyl ester (210 mg (1 mmol), chemical purity: 70.4%), (S)-α-[m-(trifluoromethyl)phenoxy]-p-chlorophenyl acetic acid (331 mg (1 mmol)) and ethyl acetate (1 mL), hexane (2 mL) was added. As a result, a solid was precipitated. The mixture was stirred at 25°C for 1 hour, and then the solid was filtered under reduced pressure. The solid was washed with hexane (2 mL), and dried in a vacuum, thereby the white solid of the title compound was obtained (yield: 102 mg, optical purity: 94.2%ee).

### Example 8: Production of (1R, 2S)-1-amino-2-vinylcyclopropanecarboxylic acid ethyl ester/(S)-N-(N-phenylcarbamoyl)-p-hydroxyphenylglycine salt;

[0063]-[0064]

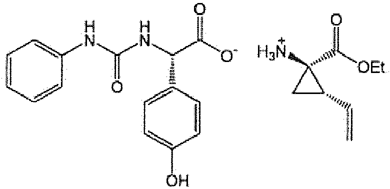

Racemic 1-amino-2-vinylcyclopropanecarboxylic acid ethyl ester (210 mg (1 mmol), chemical purity: 70.4%), (S)-N-(N-phenylcarbamoyl)-p-hydroxyphenylglycine (286 mg (1 mmol)) and ethyl acetate (2 mL) was mixed, and after a while, a solid was precipitated from the solution. The mixture was stirred at 25°C for 1 hour, and then the solid was filtered under reduced pressure. The solid was washed with hexane (2 mL), and dried in a vacuum, thereby the white solid of the title compound was obtained (yield: 190 mg, optical purity: 58.0%ee).

### INDUSTRIAL APPLICABILITY

According to the present invention, it is possible to improve the optical purity of 1-amino-2-vinylcyclopropanecarboxylic acid ester. The 1-amino-2-vinylcyclopropanecarboxylic acid can be used for producing various types of drugs.

## Claims

1. A method for producing a salt represented by the formula (3), comprising the steps of:
forming the salt represented by the following formula (3): 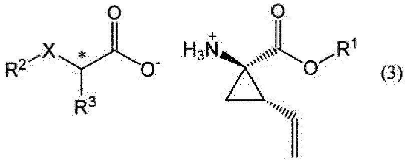 wherein, R¹ represents a C₁₋₅ alkyl group; R² represents a C₆₋₁₅ aryl group, a C₇₋₁₅ aralkyl group, a C₇₋₁₅ aroyl group or a C₇₋₁₅ N-arylcarbamoyl group, wherein the groups as R² may have one or more substiutent groups selected from an alkyl group, a halogenated alkyl group, an alkoxy group, a halo group, a nitro group, and a hydroxy group; R³ represents a C₁₋₁₅ alkyl group, a C₆₋₁₅ aryl group or a C₇₋₁₅ aralkyl group, wherein the groups as R³ may have one or more substiutent groups selected from an alkyl group, a halogenated alkyl group, an alkoxy group, a halo group, a nitro group, and a hydroxy group; X represents O, S, or NH; * indicates an asymmetric carbon atom, from (1R, 2S)-1-amino-2-vinylcyclopropanecarboxylic acid ester containing (1S, 2R)-1-amino-2-vinylcyclopropanecarboxylic acid ester as an impurity and represented by the following formula (1): 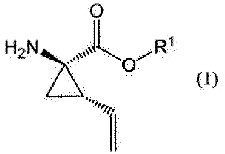 wherein, R¹ is same as the above,
and an optically active carboxylic acid represented by the following formula (2): 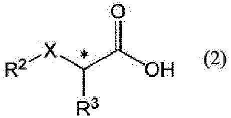 wherein, R², R³, X and * are the same as the above,
and precipitating the salt (3) from a solvent as a solid to remove the impurity.

2. The production method according to claim 1, wherein R¹ is an ethyl group.

3. The production method according to claim 1 or 2, wherein R² is a p-chlorobenzoyl group or a 3,5-dichlorobenzoyl group, R³ is a methyl group, and X is O.

4. A method for producing a compound (1) improved in optical purity,
comprising a step of dissociating the salt (3) produced by the method according to claims 1 to 3.

5. A (1R, 2S)-1-amino-2-vinylcyclopropanecarboxylic acid ester derivative represented by the following formula (3): 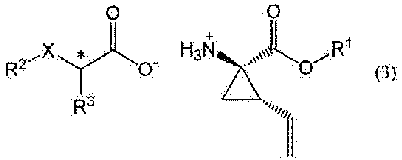 wherein R¹ represents a C₁₋₅ alkyl group; R² represents a C₆₋₁₅ aryl group, a C₇₋₁₅ aralkyl group, a C₇₋₁₅ aroyl group or a C₇₋₁₅ N-arylcarbamoyl group, wherein the groups as R² may have one or more substiutent groups selected from an alkyl group, a halogenated alkyl group, an alkoxy group, a halo group, a nitro group, and a hydroxy group; R³ represents a C₁₋₁₅ alkyl group, a C₆₋₁₅ aryl group or a C₇₋₁₅ aralkyl group, wherein the groups as R³ may have one or more substiutent groups selected from an alkyl group, a halogenated alkyl group, an alkoxy group, a halo group, a nitro group, and a hydroxy group; X represents O, S, or NH; * indicates an asymmetric carbon atom.

6. A (1R, 2S)-1-amino-2-vinylcyclopropanecarboxylic acid ester derivative according to claim 5 represented by the following formula (4): 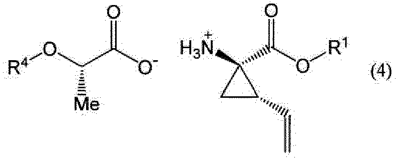 wherein R¹ represents a C₁₋₅ alkyl group; R⁴ represents a C₇₋₁₅ aroyl group.

7. The (1R, 2S)-1-amino-2-vinylcyclopropanecarboxylic acid ester according to claim 6, wherein R¹ is an ethyl group, and R⁴ is a p-chlorobenzoyl group or a 3,5-dichlorobenzoyl group.

## Patentansprüche

1. Verfahren zur Herstellung eines Salzes der Formel (3), umfassend die Schritte:
Bilden des Salzes der nachstehenden Formel (3): 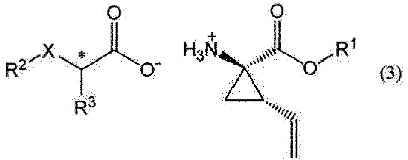 worin R¹ eine C₁₋₅-Alkylgruppe darstellt; R² eine C₆₋₁₅-Arylgruppe, eine C₇₋₁₅-Aralkylgruppe, eine C₇₋₁₅-Aroylgruppe oder eine C₇₋₁₅-N-Arylcarbamoylgruppe darstellt, wobei die R²-Gruppen eine oder mehrere Substituentengruppe(n), ausgewählt aus einer Alkylgruppe, einer halogenierten Alkylgruppe, einer Alkoxygruppe, einer Halogengruppe, einer Nitrogruppe und einer Hydroxygruppe, aufweisen können; R³ eine C₁₋₁₅-Alkylgruppe, eine C₆₋₁₅-Arylgruppe oder eine C₇₋₁₅-Aralkylgruppe darstellt, wobei die R³-Gruppen eine oder mehrere Substituentengruppe(n), ausgewählt aus einer Alkylgruppe, einer halogenierten Alkylgruppe, einer Alkoxygruppe, einer Halogengruppe, einer Nitrogruppe und einer Hydroxygruppe, aufweisen können; X O, S oder NH darstellt und * ein asymmetrisches Kohlenstoffatom bezeichnet,
aus (1R, 2S)-1-Amino-2-vinylcyclopropancarbonsäureester, enthaltend (1S, 2R)-1-Amino-2-vinylcyclopropancarbonsäureester als Verunreinigung, und der nachstehenden Formel (1): 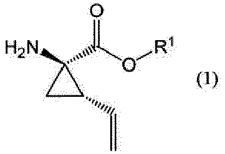 worin R¹ das gleiche wie oben ist,
und einer optisch aktiven Carbonsäure der nachstehenden Formel (2): 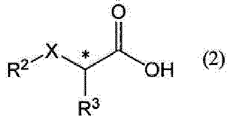 worin R², R³, X und * gleich wie oben sind,
und Ausfällen des Salzes (3) aus einem Lösungsmittel als Feststoff, um die Verunreinigungen zu entfernen.

2. Herstellungsverfahren gemäss Anspruch 1, worin R¹ eine Ethylgruppe ist.

3. Herstellungsverfahren gemäss Anspruch 1 oder 2, worin R² eine p-Chlorbenzoylgruppe oder eine 3,5-Dichlorbenzoylgruppe ist, R³ eine Methylgruppe ist und X O ist.

4. Verfahren zur Herstellung einer in der optischen Reinheit verbesserten Verbindung (1), umfassend einen Schritt, in dem das durch das Verfahren gemäss den Ansprüchen 1 bis 3 hergestellte Salz (3) dissoziiert wird.

5. (1R, 2S)-1-Amino-2-vinylcyclopropancarbonsäureester-Derivat der nachstehenden Formel (3): 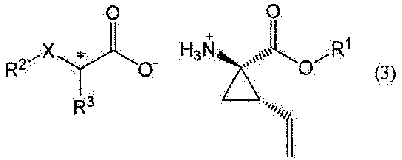 worin R¹ eine C₁₋₅-Alkylgruppe darstellt; R² eine C₆₋₁₅-Arylgruppe, eine C₇₋₁₅-Aralkylgruppe, eine C₇₋₁₅-Aroylgruppe oder eine C₇₋₁₅-N-Arylcarbamoylgruppe darstellt, wobei die R²-Gruppen eine oder mehrere Substituentengruppe(n), ausgewählt aus einer Alkylgruppe, einer halogenierten Alkylgruppe, einer Alkoxygruppe, einer Halogengruppe, einer Nitrogruppe und einer Hydroxygruppe, aufweisen können; R³ eine C₁₋₁₅-Alkylgruppe, eine C₆₋₁₅-Arylgruppe oder eine C₇₋₁₅-Aralkylgruppe darstellt, wobei die R³-Gruppen eine oder mehrere Substituentengruppe(n), ausgewählt aus einer Alkylgruppe, einer halogenierten Alkylgruppe, einer Alkoxygruppe, einer Halogengruppe, einer Nitrogruppe und einer Hydroxygruppe, aufweisen können; X O, S oder NH darstellt und * ein asymmetrisches Kohlenstoffatom bezeichnet.

6. (1R, 2S)-1-Amino-2-vinylcyclopropancarbonsäureester-Derivat der nachstehenden Formel (4) gemäss Anspruch 5: 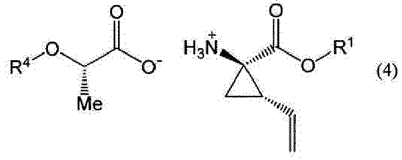 worin R¹ eine C₁₋₅-Alkylgruppe darstellt und R⁴ eine C₇₋₁₅-Aroylgruppe darstellt.

7. (1R, 2S)-1-Amino-2-vinylcyclopropancarbonsäureester gemäss Anspruch 6, worin R¹ eine Ethylgruppe ist und R⁴ eine p-Chlorbenzoylgruppe oder eine 3,5-Dichlorbenzoylgruppe ist.

## Revendications

1. Procédé de production d'un sel représenté par la formule (3), comprenant les étapes de :
formation du sel représenté par la formule suivante (3) : 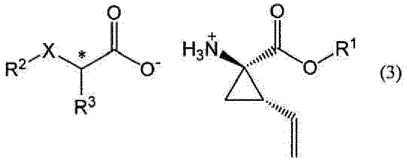 dans laquelle, R¹ représente un groupe alkyle C₁₋₅; R² représente un groupe aryle C₆₋₁₅, un groupe aralkyle C₇₋₁₅, un groupe aroyle C₇₋₁₅ ou un groupe N-arylcarbamoyle C₇₋₁₅, dans laquelle les groupes en tant que R² peuvent avoir un ou plusieurs groupes substituants choisi(s) parmi un groupe alkyle, un groupe alkyle halogéné, un groupe alkoxy, un groupe halo, un groupe nitro, et un groupe hydroxy; R³ représente un groupe alkyle C₁₋₁₅, un groupe aryle C₆₋₁₅ ou un groupe aralkyle C₇₋₁₅, dans laquelle les groupes en tant que R³ peuvent avoir un ou plusieurs groupes substituants choisi(s) parmi un groupe alkyle, un groupe alkyle halogéné, un groupe alkoxy, un groupe halo, un groupe nitro, et un groupe hydroxy; X représente O, S, ou NH ; * indique un atome de carbone asymétrique,
à partir de l'ester de l'acide (1R, 2S)-1-amino-2-vinylcyclopropanecarboxylique contenant l'ester de l'acide (1S, 2S)-1-amino-2-vinylcyclopropanecarboxylique en tant qu'impureté et représenté par la formule (1) suivante : 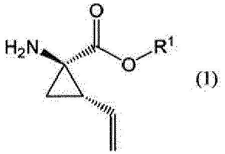 dans laquelle, R¹ est tel que défini ci-dessus,
et d'un acide carboxylique optiquement actif représenté par la formule (2) suivante : 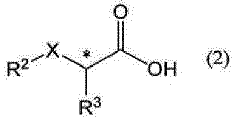 dans laquelle, R², R³, X et * sont tels que définis ci-dessus,
et la précipitation du sel (3) à partir d'un solvant en tant que solide pour retirer l'impureté.

2. Le procédé de production selon la revendication 1, dans lequel R¹ est un groupe éthyle.

3. Le procédé de production selon la revendication 1 ou 2, dans lequel R² est un groupe p-chlorobenzoyle ou un groupe 3,5-dichlorobenzoyle, R³ est un groupe méthyle, et X est O.

4. Procédé de production d'un composé (1) amélioré en pureté optique,
comprenant une étape de dissociation du sel (3) produit par le procédé selon les revendications 1 à 3.

5. Dérivé de l'ester de l'acide (1R, 2S)-1-amino-2-vinylcyclopropanecarboxylique représenté par la formule (3) suivante : 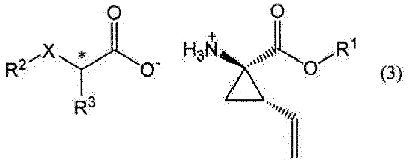 dans laquelle, R¹ représente un groupe alkyle C₁₋₅; R² représente un groupe aryle C₆₋₁₅, un groupe aralkyle C₇₋₁₅, un groupe aroyle C₇₋₁₅ ou un groupe N-arylcarbamoyle C₇₋₁₅, dans laquelle les groupes en tant que R² peuvent avoir un ou plusieurs groupes substituants choisi(s) parmi un groupe alkyle, un groupe alkyle halogéné, un groupe alkoxy, un groupe halo, un groupe nitro, et un groupe hydroxy; R³ représente un groupe alkyle C₁₋₁₅, un groupe aryle C₆₋₁₅ ou un groupe aralkyle C₇₋₁₅, dans laquelle les groupes en tant que R³ peuvent avoir un ou plusieurs groupes substituants choisi(s) parmi un groupe alkyle, un groupe alkyle halogéné, un groupe alkoxy, un groupe halo, un groupe nitro, et un groupe hydroxy; X représente O, S, ou NH ; * indique un atome de carbone asymétrique.

6. Dérivé de l'ester de l'acide (1R, 2S)-1-amino-2-vinylcyclopropanecarboxylique selon la revendication 5 représenté par la formule (4) suivante : 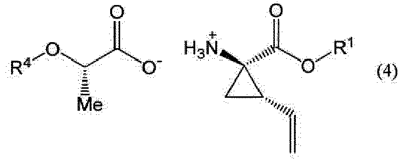 dans laquelle R¹ représente un groupe alkyle C₁₋₅ ; R⁴ représente un groupe aroyle C₇₋₁₅.

7. Ester de l'acide (1R, 2S)-1-amino-2-vinylcyclopropanecarboxylique selon la revendication 6, dans lequel R¹ est un groupe éthyle, et R⁴ est un groupe p-chlorobenzoyle ou un groupe 3,5-dichlorobenzoyle.
